# EUROPEAN PATENT SPECIFICATION

(11) **EP 3 448 341 B1**
(45) Date of publication and mention of the grant of the patent: **15.09.2021**
(21) Application number: 17722617.2
(22) Date of filing: 27.04.2017
(51) Int. Cl.: A61F 13/49, B32B 3/26, B32B 5/02, B32B 27/12

(54) **ELASTIC MATERIAL AND SANITARY ARTICLES**
ELASTISCHES MATERIAL UND HYGIENEARTIKEL
MATÉRIAU ÉLASTIQUE ET ARTICLES SANITAIRES

(30) Priority: 28.04.2016 JP 2016091516
(43) Date of publication of application: 06.03.2019
(73) Proprietor: 3M Innovative Properties Company, St. Paul, MN 55133-3427 (US)
(72) Inventor: KUNIHIRO, Kioshi, Shinagawa-ku Tokyo 141-8684 (JP)
(74) Representative: Vollmers, Hans-Gerd
(86) International application number: PCT/US2017/029900
(87) International publication number: WO 2017/189877

(56) References cited:
- WO-A1-2012/036599
- WO-A1-2015/168032
- WO-A2-2012/137084

## Description

### TECHNICAL FIELD

A first aspect of the present invention is related to elastic material. Furthermore, an additional aspect of the present invention is related to an underpants type sanitary article prepared with an elastic part

### RELATED TECHNOLOGY

An elastic material manufactured by attaching a non-woven cloth to both surfaces of an elastic film while stretching is well known (see international publication 2015/168032 pamphlet). The non-woven fabric is crimped to the elastic film such as by using spot welding.

### Related Technology Reference

Patent Document 1: International publication 2015/168032 No. pamphlet

WO2012/137084 (Kimberley Clark Co et.al.) discloses a method making an elastic nonwoven composite that contains an elastic film laminated to one or more nonwoven web materials. The method includes forming an elastic film from a polymer composition and passing the film and a nonwoven web material through a nip formed by at least one patterned roll.

WO2012/036599 (SCA Hygiene AB et.al.) discloses an elastic laminate material comprising at least one elastic film layer and at least one nonwoven layer, said elastic film layer and said nonwoven layer being bonded together in a bonding pattern comprising a plurality of bonding elements, in which said elastic film and said nonwoven are fused together, wherein the elastic film is in a stretched condition in a machine direction (MD) during bonding.

### SUMMARY OF THE INVENTION

For example, in the case that elastic material with extension processing performed is applied to a sanitary article, breathability and extendibility are important and there was room for improvement from the perspective of balancing these.

### MEANS FOR SOLVING THE VARIOUS ISSUES

An elastic material according to an aspect of the present invention comprises an elastic film, non-woven fabric laminated on both surfaces of the elastic film, and crimped parts where the non-woven fabric and elastic film are crimped and indented, the crimped part is prepared with a plurality of first emboss formed on a first line and a plurality of second emboss formed on a second line beside the first line, the first and the second line in a direction diagonally intersecting the MD direction of the elastic film, wherein the first emboss is a long lengthy shape in a direction orthogonal to the MD direction, the first line and second line are alternately arranged,
at least a part of the intervals of adjacent first emboss on the same line are smaller than the intervals of adjacent second emboss on the same line.

The elastic material is extended in the MD direction and partially cleaved ensuring breathability. Cleaving is avoided at crimped parts as cleavage would readily expand in at least the MD direction causing loss of the basic function of elasticity and so must be avoided. Here, a first emboss has a long lengthy shape in the direction orthogonal to the MD direction and furthermore, at least a part of the spacing between adjacent first emboss on the same line is smaller than the spacing between adjacent second emboss on the same line. As a result, cleavage more readily occurs between adjacent first emboss as compared to between adjacent second emboss. In other words, breathability can be ensured using cleavage in the area along a first line while cleavage does not occur readily in areas along a second line so elasticity (with suitable tension and durability) can be ensured in the area between the first line and second line. Furthermore, the first emboss and second emboss that are crimped are harder areas than other parts where if the first emboss and second emboss are, for example, aligned with the MD direction rather than in a direction intersecting the MD direction diagonally, hardness (high tension) will be readily apparent when extended in the MD direction and this can easily lead to low elasticity. On the other hand, if the first emboss and second emboss are, for example, aligned in the CD direction rather than intersecting diagonally, hardness (high tension) when extending in the CD direction will be readily apparent (to soft in MD direction lowering durability) and can easily lead to low elasticity. However, with the elastic material described above, the first line that the first emboss is lined up on and the second line that the second emboss is lined up on both intersect diagonally with the MD direction so elasticity is not easily lost and so are beneficial for providing both breathability and elasticity.

The elastic material according to a first embodiment can have a plurality of first emboss on the same line be arranged at equidistant intervals. With this type of elastic material, cleavage achieving balance between first emboss can easily be formed.

The elastic material according to the first embodiment can have a plurality of first emboss on the same line be arranged at non-equidistant intervals with interval spacing high density areas and interval spacing low density areas being formed. Cleavage is easier to achieve in the high density areas than the low density areas and cleavage in high density areas more readily coalesces and grows and so is beneficial for ensuring breathability.

Furthermore, the space between vertical emboss can bulge dramatically and this bulge can generate resistance to bending in the vertical direction and lead to reduction in softness. However, the elastic material according to this embodiment is prepared with a high density area where the bulging of a high density area is lower than that of a low density area enabling forming an area where the resistance described above is reduced. As a result, this elastic material is beneficial from the perspective of softness in addition to breathability and elasticity.

With the elastic material according to the first embodiment, the high density area can have a plurality of types with differing numbers of first emboss. With this type of elastic material, areas with varying density can be formed simplifying optimization of achieving breathability, elasticity, and softness.

With the elastic material according to the first embodiment, the second emboss can be a long lengthy shape in a direction diagonal to the MD direction. Compared to having the second emboss be a long lengthy shape in the MD direction or a long lengthy shape orthogonal to the MD direction, cleavage between second emboss occurs less readily enabling promoting effective cleavage in the area along the first line along which the first emboss are lined up.

With the elastic material described above, breathability can be ensured using cleavage in the area along a first line while cleavage does not occur readily in areas along a second line so elasticity (with suitable tension and durability) can be ensured in the area between the first line and second line. Furthermore, the first line that the first emboss is lined up on and the second line that the second emboss is lined up on both intersect diagonally with the MD direction so elasticity is not easily lost and so are beneficial for providing both breathability and elasticity.

An elastic material according to another aspect of the present invention comprises an elastic film, non-woven fabric laminated on both surfaces of the elastic film, and crimped parts where the non-woven fabric and elastic film are crimped and indented, the crimped part is prepared with a plurality of first emboss formed on a first line and a plurality of second emboss formed on a second line beside the first line in a direction diagonally intersecting the MD direction of the elastic film, wherein the first emboss is a long lengthy shape in a direction orthogonal to the MD direction, the first line and second line are alternately arranged, at least a part of the intervals of adjacent first emboss on the same line are smaller than the intervals of adjacent second emboss on the same line, and cleavage is formed on the elastic film on at least a part of the space between adjacent first emboss on the same line.

With the elastic material described above, breathability can be ensured using cleavage in the area along a first line while cleavage is difficult to generate in areas long a second line so elasticity (with suitable tension and durability) can be ensured in the area between the first line and second line. Furthermore, the first line that the first emboss is lined up on and the second line that the second emboss is lined up on both intersect diagonally with the MD direction so elasticity is not easily lost and so are beneficial for providing both breathability and elasticity.

The elastic material according to the first embodiment can have a plurality of first emboss on the same line be arranged at non-equidistant intervals with interval spacing high density areas and interval spacing low density areas being formed. Cleavage is easier to achieve in the high density areas than the low density areas and cleavage in high density areas more readily coalesces and grows and so is beneficial for ensuring breathability. Furthermore, this elastic material is prepared with a high interval density area and so is beneficial from the perspective of softness in addition to breathability and elasticity.

With the elastic material according to the first embodiment, the second emboss can be a long lengthy shape in a direction diagonal to the MD direction. Compared to having the second emboss be a long lengthy shape in the MD direction or a long lengthy shape orthogonal to the MD direction, cleavage between second emboss occurs less readily enabling promoting effective cleavage in the area along the first line along which the first emboss are lined up.

Another aspect according to the present invention is an underpants type sanitary article comprising, a main body part with an abdomen part and back part with both parts connected, the main body part being provided with a waist opening part, the main body part being provided with a pair of leg openings, and the main body part being provided with an elastic part at least along the waist opening, wherein the elastic part comprises an elastic film, non-woven fabric laminated on both surfaces of the elastic film, and crimped parts where the non-woven fabric and elastic film are crimped and indented, the crimped part is prepared with a plurality of first emboss formed on a first line and a plurality of second emboss formed on a second line beside the first line in a direction diagonally intersecting the MD direction of the elastic film, and the first emboss is a long lengthy shape in a direction orthogonal to the MD direction, the first line and second line are alternately arranged, at least a part of the intervals of adjacent first emboss on the same line are smaller than the intervals of adjacent second emboss on the same line.

With the elastic material for the sanitary article described above, breathability can be ensured using cleavage in the area along a first line while cleavage does not occur readily in areas along a second line so elasticity (with suitable tension and durability) can be ensured in the area between the first line and second line. Furthermore, the first line that the first emboss is lined up on and the second line that the second emboss is lined up on both intersect diagonally with the MD direction so elasticity is not easily lost and so are beneficial for providing both breathability and elasticity.

The elastic material for the sanitary article according to the first embodiment can have a plurality of first emboss on the same line be arranged at non-equidistant intervals with interval spacing high density areas and interval spacing low density areas being formed. Cleavage is easier to achieve in the high density areas than the low density areas and cleavage in high density areas more readily coalesces and grows and so is beneficial for ensuring breathability. Furthermore, this elastic material for sanitary articles of this embodiment is prepared with a high interval density area and so is beneficial from the perspective of softness in addition to breathability and elasticity.

### EFFECT OF THE INVENTION

An aspect of the present invention is beneficial for both breathability and elasticity.

### BRIEF DESCRIPTION OF THE DRAWINGS

Fig. 1 is a perspective drawing for elastic material according to a first embodiment.
Fig. 2 is a plan view showing the area indicated by arrow A in Fig. 1 enlarged.
Fig. 3 is a cross-sectional view along line III-III in Fig. 2.
Fig. 4 is a cross-sectional view along line IV-IV in Fig. 2
Fig. 5 is a diagram illustrating elastic material manufacturing equipment.
Fig. 6 is a plan view where elastic material according to embodiment 2 is stretched in the MD direction relative to elastic material according to embodiment 1 and shown enlarged.
Fig. 7 is a cross-sectional view along line VII-VII in Fig. 6.
Fig. 8 is a plan view where a part of elastic material according to embodiment 3 is not stretched and shown enlarged.
Fig. 9 is a plan view where a part of elastic material according to embodiment 4 is not stretched and shown enlarged.
Fig. 10 is a plan view where a part of elastic material according to embodiment 5 is not stretched and shown enlarged.
Fig. 11 is a perspective view exemplifying an underpants type sanitary article where (a) is a perspective view of a sanitary article according to embodiment 1, (b) is a perspective view of a sanitary article according to embodiment 2, and (c) is a perspective view of a sanitary article according to embodiment 3.

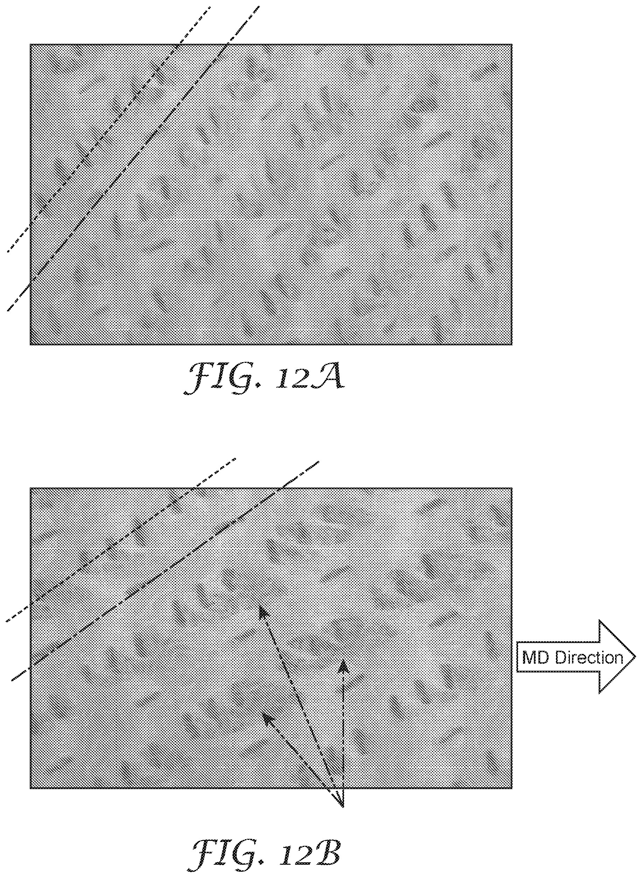

Fig. 12 is a front view of non-woven fabric according to example 1 where (a) is a picture of prior to performing extension processing showing a range of non-woven fabric of roughly 9 mm length, 15 mm width. In addition (b) is a picture of after performing extension processing showing a range of non-woven fabric of roughly 9mm length, 15 mm width. Note, the back surface drawing of non-woven fabric according to example 1 is symmetrical to the front surface drawing and so is omitted. Furthermore, the dashed line, one-dot chain line, and two-dot chain line arrows shown on the front view of example 1 are not related to design of the non-woven fabric.

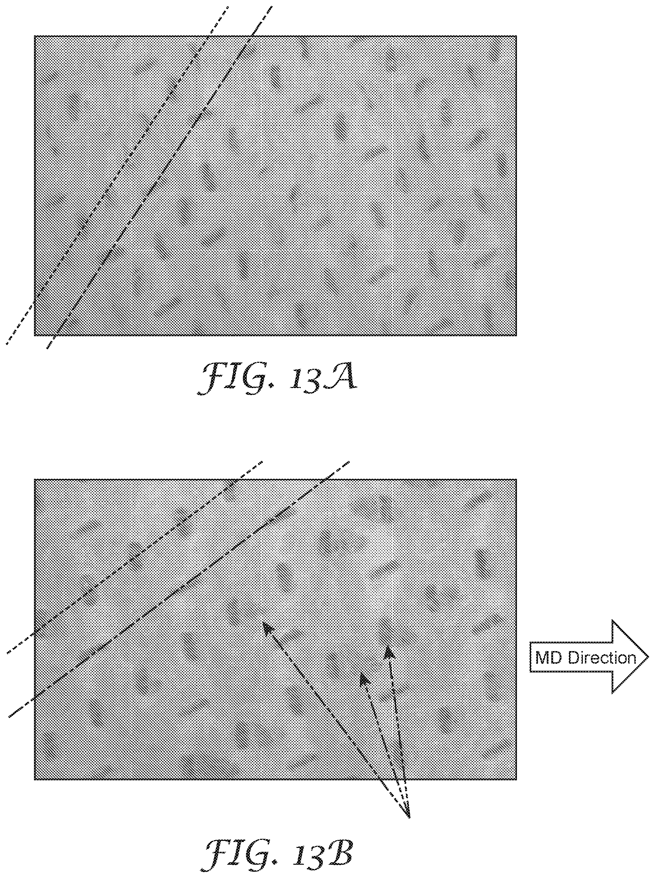

Fig. 13 is a front view of non-woven fabric according to example 2 where (a) is a picture of prior to performing extension processing showing a range of non-woven fabric of roughly 9 mm length, 15 mm width. In addition (b) is a picture of after performing extension processing showing a range of non-woven fabric of roughly 9 mm length, 15 mm width. Note, the back surface drawing of non-woven fabric according to example 2 is symmetrical to the front surface drawing and so is omitted. Furthermore, the dashed line, one-dot chain line, and two-dot chain line arrows shown on the front view of example 2 are not related to design of the non-woven fabric.

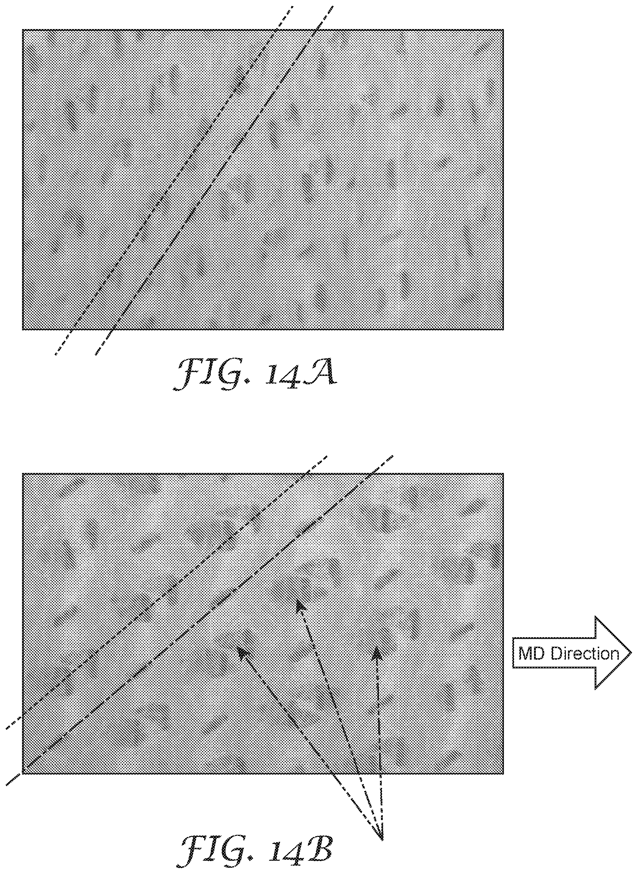

Fig. 14 is a front view of non-woven fabric according to example 3 where (a) is a picture of prior to performing extension processing showing a range of non-woven fabric of roughly 9 mm length, 15 mm width. In addition (b) is a picture of after performing extension processing showing a range of non-woven fabric of roughly 9 mm length, 15 mm width. Note, the back surface drawing of non-woven fabric according to example 1 is symmetrical to the front surface drawing and so is omitted. Furthermore, the dashed line, one-dot chain line, and two-dot chain line arrows shown on the front view of example 3 are not related to design of the non-woven fabric.

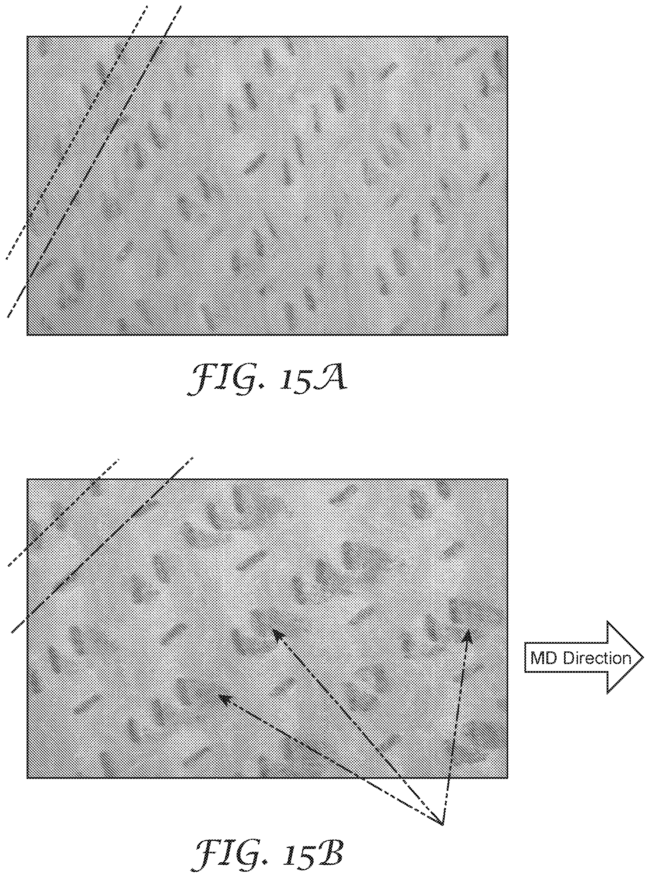

Fig. 15 is a front view of non-woven fabric according to example 4 where (a) is a picture of prior to performing extension processing showing a range of non-woven fabric of roughly 9 mm length, 15 mm width. In addition (b) is a picture of after performing extension processing showing a range of non-woven fabric of roughly 9 mm length, 15 mm width. Note, the back surface drawing of non-woven fabric according to example 1 is symmetrical to the front surface drawing and so is omitted. Furthermore, the dashed line, one-dot chain line, and two-dot chain line arrows shown on the front view of example 4 are not related to design of the non-woven fabric.
Fig. 16 is a front view of non-woven fabric according to a comparative example where (a) is a picture of prior to performing extension processing showing a range of non-woven fabric of roughly 9m length, 15 mm width. In addition (b) is a picture of after performing extension processing showing a range of non-woven fabric of roughly 9 mm length, 15 mm width.
Fig. 17 is a diagram illustrating an example of extension processing.
Fig. 18 is a diagram illustrating tension and compression tests.
Fig. 19 shows an S-S curve where (a) shows example 1 and (b) shows embodiment 2.
Fig. 20 shows an S-S curve where (a) shows example 3. (b) shows embodiment 4, and .(c) shows the comparative example.

### EMBODIMENT OF THE INVENTION

In the terms of this specification the "first line and second line being alternately arranged" is satisfied if at least a second line is arranged between adjacent first lines or a first line is arranged between adjacent second lines. Furthermore, this is not limited to the case where the first line and second line are alternately arranged over the entire area of the full surface of the elastic member but includes an aspect where the edges are excluded and only the center area has the first line and second line arranged alternately. Note, the first line and second line do not respectively need to be straight and parallel and for example, the second line can be wave shaped.

Embodiments of the present invention are described in detail with reference to attached drawings below. Note, the same codes are used for the same or equivalent elements in description of the drawings and duplicate descriptions are omitted.

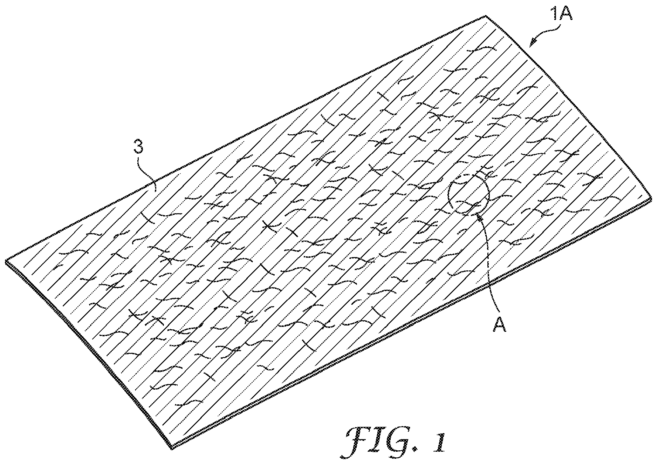

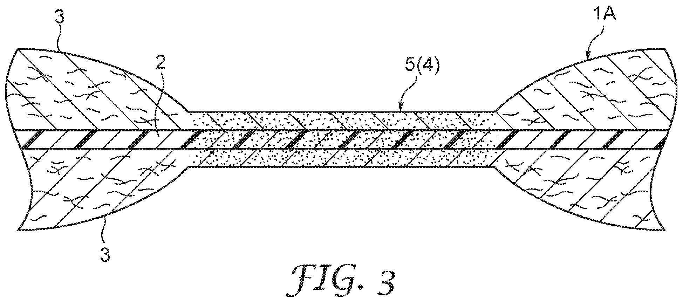

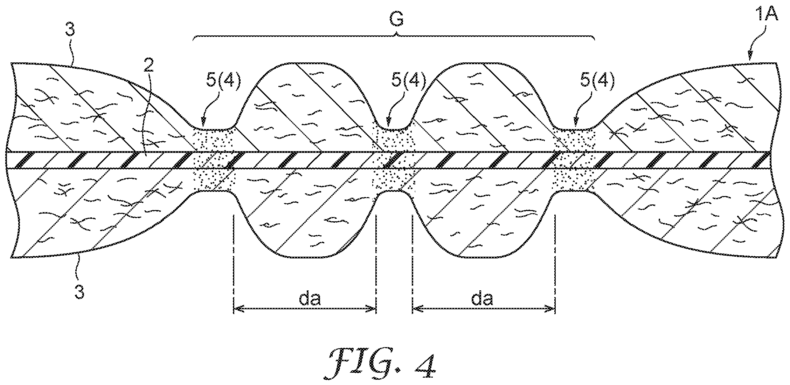

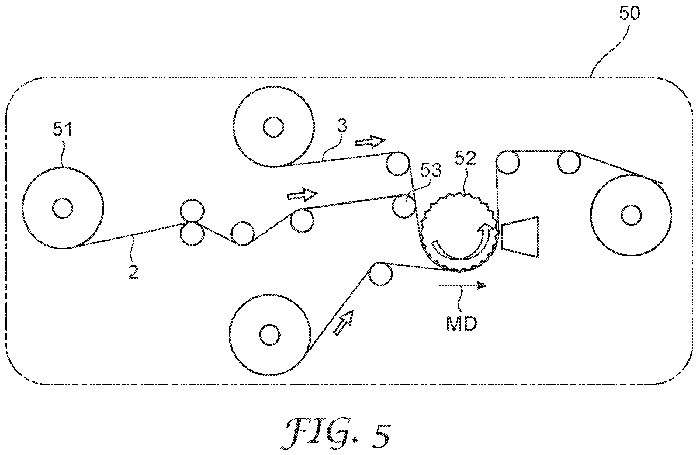

As shown in Fig. 1, Fig. 2, Fig. 3, and Fig. 4, the elastic material 1A according to embodiment 1 is a non-woven fabric material with non-woven fabric 3 laminated on both sides of an elastic film 2. When an elastic material 1A is manufactured using elastic material manufacturing equipment 50 (see Fig. 5), the elastic film 2 is provided with tension while pulling from a roll and non-woven fabric 3 is laminated on both sides of the elastic film 2. The direction that the elastic film 2 laminated with non-woven fabric 3 is fed is MD direction (Machine Direction) and the direction orthogonal to the MD direction on the surface along the elastic film 2 is the CD direction (Cross Machine Direction).

The elastic film 2 passes over a film transport roll 53, is fed along a pattern role 52 and laminated with non-woven fabric 3 here and emboss process is performed forming a prescribed crimp part 4. Here, the pattern role 52 rotates faster than the film transport roll 53 causing the elastic film 2 to be stretched in the MD direction (for example 2 to 5 times). If the elastic film 2 has tensile yield strain (if a skin-core multilayer structure, plastic deformation of the skin) this multiplication factor should be greater than the yield. With the crimp part 4, the non-woven fabric 3 is hollow and crimped to the elastic film 2 and as a result, the elastic film 2 and both sides of non-woven fabric 3 become integrated and form the elastic material 1A.

The crimp part 4 crimps the non-woven fabric 3 and elastic film 2 and is provided with a plurality of hollow embossed parts. The plurality of embossed parts can basically be classified into two types of conditions. Specifically this is a vertical emboss 5 long and lengthy in the CD direction and non-vertical emboss 6 other than the vertical emboss 5. The dimension in the longitudinal direction of the vertical emboss 5 is in the range of 0.1 mm to 10 mm. Furthermore, the vertical emboss 5 is an example of a first emboss and is a long groove shape in the present embodiment. However, it is sufficient for the vertical emboss 5 to be specified as a long and lengthy shape in the CD direction for instance an elliptical shape, rectangular shape, or other shape.

Furthermore, the non-vertical emboss 6 is an example of the second emboss and in the present embodiment is a long grove with a long and lengthy shape in the direction intersecting diagonally with the MD direction. The dimension in the longitudinal direction of the non-vertical emboss 6 is in the range of 0.1 mm to 10 mm. Furthermore, the non-vertical emboss 6 can be an elliptical shape, rectangular shape, or other shape as long as it is not long and lengthy in the MD direction or CD direction.

Non-woven fabric 3 is laminated on both surfaces of the elastic material 1A. When viewing the surface of the non-woven fabric 3, a plurality of vertical emboss 5 are formed in the first line lined up in the direction intersecting diagonally relative to the MD direction and a plurality of first lines are formed on the surface of the non-woven fabric 3 without differentiating front and back. When identifying the first line, for example, one of the vertical emboss 5 are designated as reference. Next, the closest adjacent vertical emboss 5 to the reference vertical emboss 5 is specified and straight lines are suppositioned in the direction these two vertical emboss 5 are lined up with. Note that in the case a plurality of vertical emboss are lined up in a wave shape, the lineup direction is taken to be a straight line that connects between the amplitude (center point) of the wave shapes. Next, if a plurality of other vertical emboss 5 are arranged overlapped on this straight line, the first line is judged to be formed by this plurality of vertical emboss 5 lined up on this straight line.

Furthermore, the plurality of non-vertical emboss 6 form a second line lined up in a direction diagonally intersecting relative to the MD direction. The surface of the non-woven fabric 3 has a plurality of second lines formed and the second lines are respectively formed along the first line. Specifically, if the plurality of non-vertical emboss 6 provided along the first line exist, the second line is made up of these plurality of non-vertical emboss 6.

Here, a "direction intersecting diagonally relative to the MD direction" means not being a direction in the MD direction (Machine Direction) or a direction in the CD direction (Cross Machine Direction) that is orthogonal to the MD direction. Specifically describing this, a direction that diagonally intersects relative to the MD direction is a direction 10 degrees to 80 degrees, preferably 15 degrees to 75 degrees, and more preferably 20 degrees to 60 degrees relative to the MD direction. In further detail, prior to extension processing and under tension (when performing emboss processing using the elastic material manufacturing equipment 50); preferably 10 degrees to 50 degrees relative to the MD direction, prior to extension processing and released from the elastic material manufacturing equipment 50 and loosened; preferably 35 degrees to 75 degrees relative to the MD direction, after extension processing and while under tension (while performing cleavage); preferably 15 degrees to 55 degrees relative to the MD direction, after extension processing and loosening after performing extension processing, preferably 40 degrees to 80 degrees relative to the MD direction.

A plurality of first lines and plurality of second lines are alternately arranged. The being alternately arranged is satisfied if at least a second line is arranged between adjacent first lines or a first line is arranged between adjacent second lines. However, this is not limited to the case that a plurality of first lines and plurality of second lines being alternately arranged over the entire surface of the elastic material 1A but first lines can be lined up on a part or second lines can be lined up on a part.

A plurality of emboss 5 on the same line can be arranged at non-equidistant intervals with interval spacing high density areas Sa and interval spacing low density areas Sb being formed. Specifically, with the present embodiment, three vertical emboss 5 are lined up forming one emboss group G and a plurality of emboss groups G are lined up on the first line. The interval between a plurality of emboss group G (hereafter "low density intervals") db is larger than the interval between three vertical emboss 5 (hereafter "high density interval") that form an emboss group G. In other words, areas that are emboss group G are high density areas Sa and areas between emboss group G are low density areas Sb.

On the other hand, the intervals between non-vertical emboss 6 are the same. The intervals for non-vertical emboss 6 are at least larger than the vertical emboss 5 high density interval da. This means that at least a part of the intervals (high density intervals) between vertical emboss 5 lined up on the same line are smaller than the intervals of non-vertical emboss 6.

Note, the intervals for vertical emboss 5 and intervals for non-vertical emboss 6 basically mean the distance in the direction along line one or line two. In other words, when looking at two vertical emboss 5 lined up next to each other along line 1, the closest distance can be taken to be the interval between vertical emboss 5. Furthermore, when looking at two non-vertical emboss 6 lined up next to each other along line 2, the closest distance can be taken to be the interval between non-vertical emboss 6.

When considering the arrangement relationship of the vertical emboss 5 and non-vertical emboss 6, the small interval part (high density part) between vertical emboss 5 adjacent on the same line is smaller than the interval with a non-vertical emboss 6. Specifically, with an arbitrary vertical emboss 5 designated as reference, of two vertical emboss 5 adjacent to the reference vertical emboss 5 (reference emboss), specify the vertical emboss 5 (adjacent emboss) with the smaller interval. Here, the interval between the adjacent emboss and the reference emboss is smaller than the interval of the non-vertical emboss 6 closest to the reference emboss with the reference emboss.

Note, the interval of vertical emboss 5 is the interval between vertical emboss 5 on the same line and means the closest position of the vertical emboss 5 and in the present embodiment is a distance along the first line. The intervals of vertical emboss 5 of the high density areas Sa can be 0.1 mm to 3 mm and preferably are 0.3 mm to 1 mm. The intervals of vertical emboss 5 of the low density areas Sb are larger than the high density areas Sa and can be 0.5 mm to 20 mm and preferably are 0.8 mm to 3 mm. Furthermore, with the present embodiment, each of the high density interval da are uniform and the low density interval db are also uniform but these do not have to strictly be uniform. Furthermore, by there being both high density interval da and low density interval db, overall, the interval of a plurality of vertical emboss 5 are non-uniform.

Furthermore, according to the present embodiment, the elastic material 1A has non-woven fabric 3 laminated on both surfaces and the natural softness of the material is not readily lost due to forming of crimp part 4. This softness can be shown using a Gurley stiffness value and means, for example, the lowness of rigidity when bent in a vertical direction relative to the surface thereof (vertical direction from either MD direction or CD direction). For example, the non-woven fabric 3 bulges between vertical emboss 5 generating a mountain, hill, or fold type bulge. This bulge part generates resistance relative to bending in the vertical direction and in particular if this bulge part is formed over the entire elastic material 1A, it may lead to reducing the level of softness. However, the elastic material 1A according to this embodiment is prepared with an interval spacing high density areas Sa and interval spacing low density areas Sb where the bulging of a high density area Sa is lower than that of a low density area Sb enabling forming an area where the resistance described above is reduced. As a result, this elastic material 1A is better from the perspective of softness. Furthermore, the first line on which a plurality of vertical emboss 5 are lined up on is along a direction diagonally intersecting relative to the MD direction where even if there is a bulge part formed, the bulge part will be formed along a direction intersecting diagonally relative to the MD direction. As a result, this does not readily cause resistance for a position along the MD direction or CD direction and is therefore more beneficial from the perspective of softness in the MD direction and CD direction.

The elastic film 2 is made up of a resin material containing elastomer. The type of elastomer is not in particular restricted and examples are styrene-isoprene-styrene block copolymer (SIS), styrene-butadiene-styrene block copolymer (SBS), styrene-ethylene/butylene-styrene block copolymer (SEBS), polyurethane, ethylene copolymer (for example ethylene vinyl acetate, ethylene propylene copolymer, ethylene propylene diene terpolymer), and propylene oxide (PO). From the perspective of convenience during the manufacturing process, a multilayer structure with a polyolefin or the like skin on at least one surface of an elastomer core is preferable.

Resin material that makes up the elastic film 2 preferably contains components other than those described above. For example this resin material preferably contains a stiffening agent (for example polyvinyl styrene, polystyrene, poly α-methyle styrene, polyester, epoxy resin, polyolefin, coumarone-indene resin), a viscosity reducing agent, plasticizer, tackifier (for example, aliphatic hydrocarbon tackifier, aromatic hydrocarbon tackifier, terpene resin tackifier, hydrogenated terpene resin tackifier), dyes, pigments, antioxident, antistatic agent, adhesive, antiblocking agent, slip agent, maturation stabilizer, light stabilizer, foaming agent, glass bubble, starch, metal salt, and micro fibers and the like.

The non-woven fabric 3 can be widely used as a material machined into fibers where materials can be suitably combined or fiber length or fiber size adjusted to provided a prescribed level of softness. The material of the non-woven fabric 3 is preferably a mixed fiber of, for example, polypropylene fiber, polyester fiber, and polyolefin fiber blended, a concentric composite fiber of a polyethylene terephthalate core material with polyethylene covering the core material. The manufacturing method of the non-woven fabric 3 is preferably, for example, the spun bond method, spun lace method, or thermal bond method.

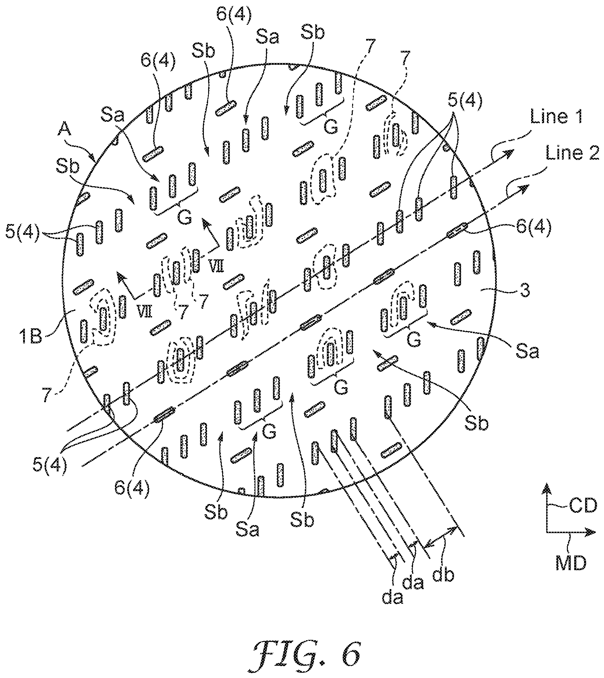

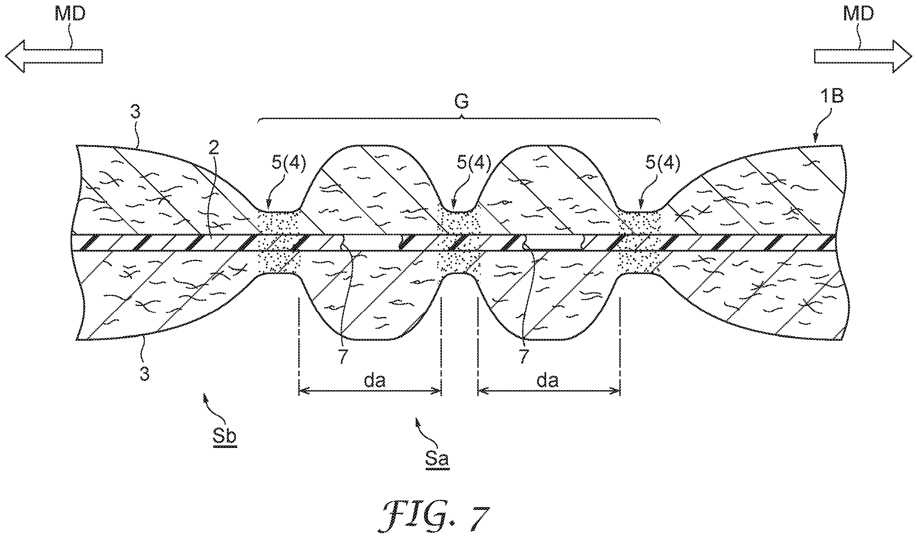

Next, elastic material 1B according to embodiment 2 will be described with reference to Fig. 6 and Fig. 7. The elastic material 1B according to the embodiment 2 is manufactured by performing extension processing in the MD direction with regards to the elastic material 1A of embodiment 1. The extension processing extends at least a part of the pre-extended elastic material 1A and is formed generating cleavage 7 for breathability in the elastic film 2. The method of extending the elastic material 1B is not in particular restricted where, for example, forming can be performed by clamping both ends of the elastic material 1B prior to extending at a prescribed width and then extending.

Temperature conditions when extending the elastic material 1B are not in particularly restricted and can be at room temperature. The extension multiple of the elastic material 1B should be roughly 1.5 to 3 times compared to the elastic material 1A loosened after manufacturing on the elastic material manufacturing equipment 50 (equivalent to 2 to 5 times that of the elastic film 2 prior to manufacturing the elastic material 1A) and can be greater than the extension multiple assumed for practicality.

Performing extension processing on the elastic material 1B according to embodiment 1 causes cleavage 7 to be generated on the elastic film 2 and as a result suitable breathability is ensured. The cleavage 7 is generated outside of the vertical emboss 5 and non-vertical emboss 6. Here, the vertical emboss 5 have a long length shape in the direction orthogonal to the MD direction and the intervals between adjacent vertical emboss 5 on the same line are smaller than the intervals between adjacent non-vertical emboss 6 on the same line. As a result, cleavage more readily occurs between adjacent vertical emboss 5 as compared to between adjacent non-vertical emboss 6.

In other words, with the elastic material 1B according to embodiment 2, cleavage 7 is formed in the elastic film 2 and the cleavage 7 is generated in the area along line 1. In Fig. 6, a plurality of locations where cleavage 7 have been generated are shown using dashed lines but in reality, cleavage 7 are generated in even more locations.

If extension processing is performed in the MD direction, stress more readily concentrates in the high density areas Sa rather than low density areas Sb and thus cleavage 7 are more likely to be generated in high density areas Sa. Furthermore, the intervals between high density areas Sa in other words between vertical emboss 5 that form an emboss group G are narrow causing aggregation of cleavage 7 that occur separately causing larger cleavage 7 to more readily grow. As a result this can be said to be beneficial for improving breathability.

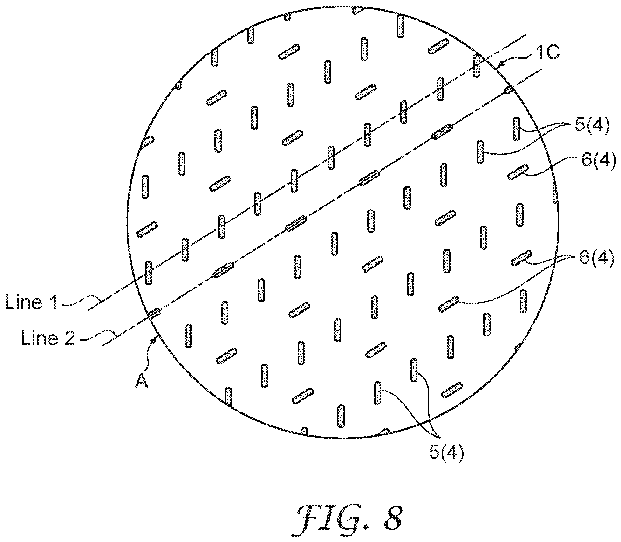

Next, elastic material 1C according to embodiment 3 will be described with reference to Fig. 8. Note, with regards to the elastic material 1C according to embodiment 3, the same codes are used for elements and structures similar to those of elastic material 1A according to embodiment 1 and a detailed description is omitted.

The elastic material 1C according to embodiment 3 is a non-woven fabric material with non-woven fabric 3 laminated on both sides of an elastic film 2. The elastic material 1C is prepared with crimp parts 4 crimped to the elastic film 2 where the non-woven fabric 3 is hollow and is integrated with the elastic film 2 and non-woven fabric 3 on both surfaces by the crimp part 4.

The crimp part 4 is provided with a plurality of vertical emboss 5 and plurality of non-vertical emboss 6. The plurality of vertical emboss 5 form a first line lined up in a direction diagonally intersecting relative to the MD direction. The surface of the non-woven fabric 3 has a plurality of first lines formed. Furthermore, the plurality of non-vertical emboss 6 form a second line lined up in a direction diagonally intersecting relative to the MD direction. The surface of the non-woven fabric 3 has a plurality of second lines formed and the second lines are respectively formed along the first line. A plurality of first lines and plurality of second lines are alternately arranged. Furthermore, the intervals between adjacent vertical emboss 5 on the same line are smaller than the intervals between adjacent non-vertical emboss 6 on the same line.

The intervals of a plurality of vertical emboss 5 lined up on the same line are the same (equidistant intervals) and the intervals of a plurality of non-vertical emboss 6 lined up on the same line are the same (equidistant intervals). In other words, with the elastic material 1C of the present embodiment, high density areas Sa and low density areas Sb are not formed. Note, judgment of whether or not intervals are equidistant is that there is not variability between the intervals of the plurality of vertical emboss 5 and the intervals of the non-vertical emboss 6 and if there is error of roughly 1 mm between the widest interval and the narrowest interval, they are judged to be equidistant intervals.

Performing of extension processing in the MD direction with regards to the elastic material 1C according to embodiment 3 provides post extension processed elastic material 1C. With the post extension processed elastic material 1C, cleavage 7 is formed in the elastic film 2 and the cleavage 7 is generated in the area along line 1. With the present embodiment, the intervals of the plurality of vertical emboss 5 lined up on the same line are the same so there is no difference in density causing cleavage 7 to occur relatively uniform.

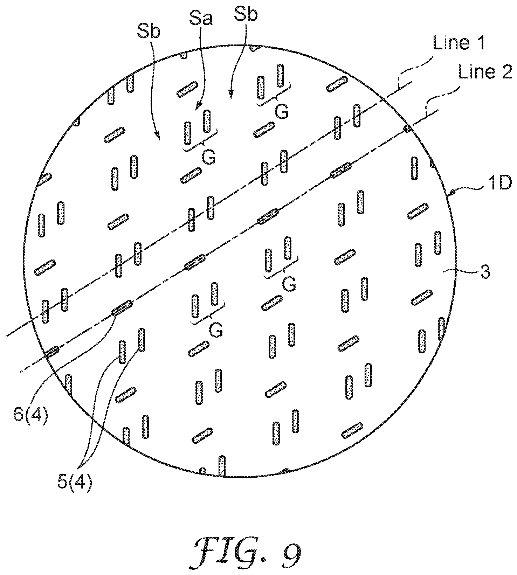

Next, elastic material ID according to embodiment 4 will be described with reference to Fig. 9. Note, with regards to the elastic material ID according to embodiment 4, the same codes are used for elements and structures similar to those of elastic material 1A according to embodiment 1 and a detailed description is omitted.

The elastic material ID according to embodiment 4 is a non-woven fabric material with non-woven fabric 3 laminated on both sides of an elastic film 2. The elastic material ID is prepared with crimp parts 4 crimped to the elastic film 2 where the non-woven fabric 3 is hollow and is integrated with the elastic film 2 and non-woven fabric 3 on both surfaces by the crimp part 4.

The crimp part 4 is provided with a plurality of vertical emboss 5 and plurality of non-vertical emboss 6. The plurality of vertical emboss 5 form a first line lined up in a direction diagonally intersecting relative to the MD direction. The surface of the non-woven fabric 3 has a plurality of first lines formed. Furthermore, the plurality of non-vertical emboss 6 form a second line lined up in a direction diagonally intersecting relative to the MD direction and the surface of the non-woven fabric 3 has a plurality of second lines formed and the second lines are respectively formed along the first line. A plurality of first lines and plurality of second lines are alternately arranged.

In the case of the elastic material ID according to embodiment 4, there are two vertical emboss 5 that form the emboss group G and a plurality of emboss group G are lined up on line 1. The intervals between a plurality of emboss group G (low density intervals) are larger than the intervals of the two vertical emboss 5 (high density intervals). Areas that are emboss group G are high density areas Sa and areas between emboss group G are low density areas Sb. Furthermore, the intervals between adjacent vertical emboss 5 on the same line are at least smaller than a part of (dense intervals) the intervals between adjacent non-vertical emboss 6 on the same line.

Performing of extension processing in the MD direction with regards to the elastic material ID according to embodiment 4 enables forming post extension processed elastic material 1D. With the post extension processed elastic material ID, cleavage 7 is formed in the elastic film 2 and the cleavage 7 is generated in the area along line 1. Furthermore, the intervals between high density areas Sa in other words between vertical emboss 5 that form an emboss group G are narrow causing aggregation of cleavage 7 that occur separately causing larger cleavage 7 to more readily grow.

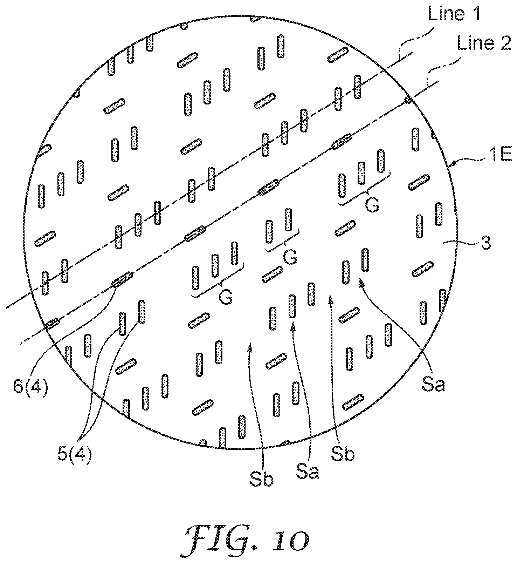

Next, elastic material IE according to embodiment 5 will be described with reference to Fig. 10. Note, with regards to the elastic material IE according to embodiment 5, the same codes are used for elements and structures similar to those of elastic material 1A according to embodiment 1 and a detailed description is omitted.

The elastic material IE according to embodiment 5 is a non-woven fabric material with non-woven fabric 3 laminated on both sides of an elastic film 2. The elastic material IE is prepared with crimp parts 4 crimped to the elastic film 2 where the non-woven fabric 3 is hollow and is integrated with the elastic film 2 and non-woven fabric 3 on both surfaces by the crimp part 4.

The crimp part 4 is provided with a plurality of vertical emboss 5 and plurality of non-vertical emboss 6. The plurality of vertical emboss 5 form a first line lined up in a direction diagonally intersecting relative to the MD direction. The surface of the non-woven fabric 3 has a plurality of first lines formed. Furthermore, the plurality of non-vertical emboss 6 form a second line lined up in a direction diagonally intersecting relative to the MD direction. The surface of the non-woven fabric 3 has a plurality of second lines formed and the second lines are respectively formed along the first line. A plurality of first lines and plurality of second lines are alternately arranged.

The elastic material IE according to embodiment 5 is a hybrid of embodiment 1 and embodiment 3 where forming two types of emboss group G provides two types of high density areas Sa. Specifically being provided with a first emboss group G made up of three vertical emboss 5 and a second emboss group G being provided with two vertical emboss 5.

The first emboss group G and second emboss group G are arranged alternately on the same line; however, the intervals of the emboss group G are not uniform; there being locations where the interval is wide and locations where the interval is narrow. In other words, with regards to the high density areas Sa of the elastic material IE, there are a plurality of different types of vertical emboss 5 enabling forming various high density areas Sa.

On the other hand, the intervals between the three vertical emboss 5 that form the first emboss group G and the intervals between the two vertical emboss 5 that form the second emboss group G (high density intervals) are uniform. Areas that are the first emboss group G or second emboss group G are high density areas Sa and areas between emboss group G are low density areas Sb. Furthermore, the intervals between adjacent vertical emboss 5 on the same line are at least smaller than a part of (dense intervals) the intervals between adjacent non-vertical emboss 6 on the same line.

Performing of extension processing in the MD direction with regards to the elastic material IE according to embodiment 5 enables forming post extension processed elastic material IE. With the post extension processed elastic material IE, cleavage 7 is formed in the elastic film 2 and the cleavage 7 is generated in the area along line 1. Furthermore, the intervals between high density areas Sa in other words between vertical emboss 5 that form an emboss group G are narrow causing aggregation of cleavage 7 that occur separately causing larger cleavage 7 to more readily grow.

With the elastic material 1A, 1C to IE of embodiments 1, 3 to 5 described above being extended in the MD direction enables locational generation of cleavage 7 ensuring breathability as in elastic material 1B according to embodiment 2. Cleaving is avoided at crimped parts as cleavage would readily expand in at least the MD direction causing loss of the basic function of elasticity and so must be avoided. Here, the vertical emboss 5 have a long length shape in the direction orthogonal to the MD direction and the intervals between at least a part of adjacent vertical emboss 5 on the same line are smaller than the intervals between adjacent non-vertical emboss 6 on the same line. As a result, cleavage more readily occurs between adjacent vertical emboss 5 as compared to between adjacent non-vertical emboss 6, elasticity (with suitable tension and durability) can be ensured in areas between the first line and second line. Furthermore, the vertical emboss 5 and non-vertical emboss 6 that are crimped are harder areas than other parts where if the vertical emboss 5 and non-vertical emboss 6 are, for example, aligned with the MD direction rather than in a direction intersecting the MD direction diagonally, hardness (high tension) will be readily apparent when extended in the MD direction and this can easily lead to low elasticity. On the other hand, if the vertical emboss and non-vertical emboss are, for example, aligned in the CD direction rather than intersecting diagonally, hardness (high tension) when extending in the CD direction will be readily apparent (to soft in MD direction lowering durability) and can easily lead to low elasticity. However, with the elastic material 1 to IE described above, the first line that the vertical emboss 5 is lined up on and the second line that the non-vertical emboss 6 is lined up on both intersect diagonally with the MD direction so elasticity is not easily lost and so are beneficial for providing both breathability and elasticity.

Furthermore, with the elastic material 1C according to embodiment 3, a plurality of vertical emboss 5 are arranged on the same line so with regards to this elastic material 1C, cleavage 7 is readily formed in a balanced manner between the vertical emboss 5.

With the elastic materials 1A, 1B, ID, IE of embodiments 1, 2, 4, and 5, a plurality of vertical emboss 5 on the same line are formed at non-equidistant intervals with interval spacing high density areas Sa and interval spacing low density areas Sb being formed. Cleavage 7 is easier to achieve in the high density areas Sa than the low density areas Sb and cleavage 7 in high density areas Sa more readily coalesce and grow and so is beneficial for ensuring breathability. Furthermore, the space between vertical emboss can bulge dramatically and this bulge can generate resistance to bending in the vertical direction and lead to reduction in softness. However, the elastic materials 1A, 1B, ID, IE are prepared with an interval spacing high density areas Sa where the bulging of a high density area Sa is lower than that of a low density area Sb enabling forming an area where the resistance described above is reduced. As a result, these elastic materials 1A, 1B, ID, IE are beneficial from the perspective of softness in addition to breathability and elasticity.

Furthermore, with the elastic material IE according to embodiment 5, there are a plurality of types of high density areas Sa with differing number of vertical emboss 5 and with this type of elastic material IE, various high density areas Sa can be formed simplifying optimization of achieving breathability, elasticity, and softness.

Furthermore, with the elastic material 1A to IE according to embodiments 1 to 5, the non-vertical emboss 6 can be a long lengthy shape in a direction diagonal to the MD direction. Compared to having the non-vertical emboss 6 be a long lengthy shape in the MD direction or a long lengthy shape orthogonal to the MD direction, with the elastic material 1A to IE of the embodiments described above, cleavage between non-vertical emboss 6 occurs less readily enabling promoting effective cleavage in the area along the first line along which the vertical emboss 5 are lined up.

Next, sanitary articles that apply the elastic material 1A to IE according to each of the embodiments described above are described. The sanitary articles can be widely applied to underpants shape and can be applied to disposable diapers used for infants, disposable diapers used for adults, and for sanitary napkins.

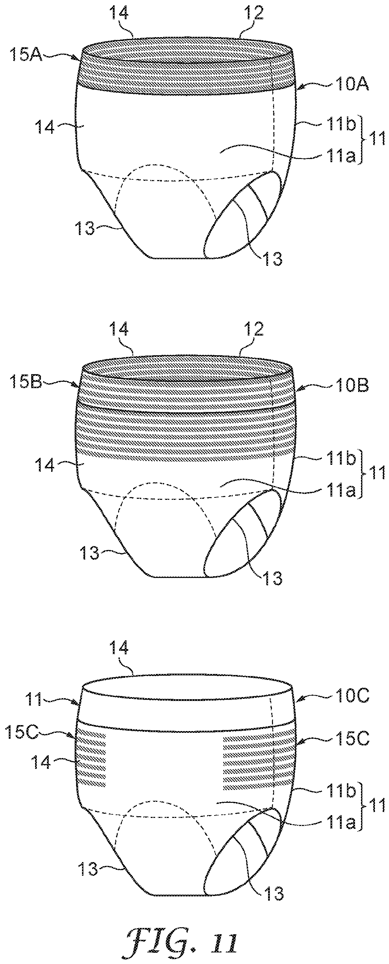

Fig. 11 is a drawing illustrating underpants type sanitary articles 10A, 10B, 10C such as, for example, disposable diapers. Underpants type is a form distinguishable from open type closed with tape having a main body part 11 that connects between an abdomen part 11a and back part 11b, a waist opening part 12 provided on this main body 11, and a pair of leg openings 13 provided on this main body 11. The main body part 11 comprises a ring shaped waist part 14 and mount part 15 that covers the lower abdomen, crotch, and buttocks. The waist part 14 is a ring shaped part provided along the waist opening part 12 and the pair of leg openings 13 are formed in the mount part 15.

In order to improve fit to the body elastic parts 15A, 15B, 15C are arranged and secured in suitable locations. The elastic parts 15A, 15B, 15C are formed by attaching the elastic materials 1A to IE according to each of the embodiments described above. There are no restrictions in particular regarding attaching of elastic materials 1A to IE and, for example, adhesive such as hot melt adhesive or sonic bond can be used. Note, in Fig. 11 the areas where elastic parts 15A, 15B, 15C are formed are indicated using a horizontal striped pattern for convenience.

Fig. 11 (a) is a sanitary article according to embodiment 1 with an elastic part 15A provided on the waist part 14 of the main body part 11 and as a result, the elastic part 15A is provided along the full circumference of the waist opening part 12. Furthermore, Fig. 11 (b) is a sanitary article according to embodiment 2 and elastic part 15B is provided along the entire circumference of the waist opening part 12. Furthermore, with this embodiment, the elastic part 15B is provided on a part of the waist part 14 and mount part 15 of the main body part 11 but can be provided on all rather than just a part of the mount part 15. For example, in Fig. 11 (b), the elastic part 15B is not provided up to the leg opening part 13 but can be provided up to the leg opening part 13. Furthermore, not providing on the waist part 14 and providing on part or all of the mount part 15 is feasible. Fig. 11 (c) is a sanitary article according to embodiment 3 with an elastic part 15C provided on both left and right sides of the mount part 15 of the main body part 11. This is because there are cases where it is preferable that the location where the sanitary article absorbent body is provided does not stretch.

Note, in the case that the elastic material 1A according to embodiment 1 described above or elastic material 1C, ID, IE according to embodiments 3 to 5 are applied as the elastic parts 15A, 15B, 15C described above, extension processing is performed for the elastic parts 15A, 15B, 15C of the sanitary articles 10A, 10B, 10C and can be attached after forming cleavage 7 for breathability with regards to the elastic film 2 of the elastic parts 15A, 15B, 15C or after attaching the elastic material 1A, 1C, ID, IE, cleavage can be formed through stretching to the wearer when putting on the sanitary article.

For example, in the case of applying the first emboss or second emboss to the periphery of the waist or buttocks of sanitary articles of a diaper prepared with an elastic part lined up in the MD direction of the first emboss and second emboss, the sanitary article will not widen out (not stretch) when being put on and therefore be more difficult to put on. For example, if the first emboss or second emboss is applied to the periphery of the waist or buttocks of a sanitary article such as a diaper prepared with an elastic part lined up in the CD direction, they are unable to follow the movements of the wearer after the sanitary article is put on causing the sanitary article to stick or gaps between the sanitary article and the body lowering the feeling of fit.

However, with the sanitary articles 10A, 10B, 10C prepared with elastic parts 15A, 15B, 15C, the line 1 that the vertical emboss 5 are lined up on and line 2 that the non-vertical emboss 6 are lined up on both diagonally intersect the MD direction and so are beneficial with regards to elasticity. In other words, sanitary articles 10A, 10B, 10C prepared with elastic parts 15A, 15B, 15C are beneficial from the perspective of both breathability and elasticity. Furthermore, in the case that the elastic material 1A, 1B, ID, IE described above are applied as the elastic parts 15A, 15B, 15C, they are beneficial from the perspective of softness in addition to breathability and elasticity.

### EXAMPLES

The present invention is described in further detail below using examples but the present invention is not restricted to these examples. Note, polypropylene spun bond non-woven fabric 15 g/sqm was used for all of the examples. A three layer film that is a 35 g/sqm skin-core-skin was used as the elastic film that is the elastic material raw material and polypropylene was used for each skin and SIS type was used as the core.

### [Example-1]

Fig. 12 is a front view that shows non-woven fabric material according to example 1 where (a) is a picture of non-woven fabric material prior to performing extension processing and in a loose state and (b) is a picture of non-woven fabric material after performing extension processing and in a stretched state. Note, the dashed line in the front view shows the first line for convenience, the one dot chain line shows the second line for convenience and further, the arrow shown by the two dot chain line indicates a part of cleavage. The dashed line, one-dot chain line, and two-dot chain line arrow are not related to design of the non-woven fabric material. Furthermore, the back surface drawing of non-woven fabric material according to example-1 is symmetrical to the front surface drawing and is therefore omitted.

The non-woven fabric material according to example-1 is a non-woven fabric material with non-woven fabric laminated on both surfaces of an elastic film and emboss forming performed to form crimped parts. The surface of the non-woven fabric material has a plurality of vertical emboss and plurality of non-vertical emboss formed. The non-woven fabric material according to example-corresponds to the elastic material according to example-1 and to the elastic material according to example-2. In other words, three vertical emboss are lined up in a dense manner forming one emboss group and a plurality of emboss groups are lined up on the first line. The intervals between a plurality of emboss group (low density intervals) are larger than the intervals of the three vertical emboss 5 (high density intervals) that form the emboss group. Areas that are emboss group are high density areas and areas between emboss group are low density areas.

The dimension in the longitudinal direction of the vertical emboss is approximately 1 mm and width is approximately 0.2 mm. The dimension in the longitudinal direction of the non-vertical emboss is approximately 1 mm and width is approximately 0.2 mm. Furthermore, the high density interval of vertical emboss is approximately 0.7 mm and low density interval is approximately 2 mm. Furthermore, the interval of non-vertical emboss lined up on the same line is larger than the high density interval of vertical emboss and is approximately 4 mm. Furthermore, the thickness of the non-woven fabric material according to example-1 is 1 mm.

### [Example-2]

Fig. 13 is a front view that shows non-woven fabric material according to example 2 where (a) is a picture of non-woven fabric material prior to performing extension processing and in a loose state and (b) is a picture of non-woven fabric material after performing extension processing and in a stretched state. Note, the dashed line in the front view shows the first line for convenience, the one dot chain line shows the second line for convenience and further, the arrow shown by the two dot chain line indicates a part of cleavage. The dashed line, one-dot chain line, and two-dot chain line arrow are not related to design of the non-woven fabric material. Furthermore, the back surface drawing of non-woven fabric material according to example-2 is symmetrical to the front surface drawing and is therefore omitted.

The non-woven fabric material according to example-2 is the same as the non-woven fabric material according to example-1 except for the shape, size, and placement of the plurality of vertical emboss and plurality of non-vertical emboss. The non-woven fabric material according to example-2 corresponds to the elastic material according to embodiment 3 described above. In other words, the plurality of vertical emboss formed on one line are lined up uniformly.

The dimension in the longitudinal direction of the vertical emboss is approximately 1 mm and width is approximately 0.3 mm. The dimension in the longitudinal direction of the roughly non-vertical emboss is approximately 1 mm and width is approximately 0.3 mm. Furthermore, the interval of the vertical emboss on the same line is approximately 2 mm and the interval of the non-vertical emboss on the same line is larger than the interval of the vertical emboss and is approximately 4 mm. Furthermore, the thickness of the non-woven fabric material according to example-2 is 1.9 mm.

### [Example-3]

Fig. 14 is a front view that shows non-woven fabric material according to example 3 where (a) is a picture of non-woven fabric material prior to performing extension processing and in a loose state and (b) is a picture of non-woven fabric material after performing extension processing and in a stretched state. Note, the dashed line in the front view shows the first line for convenience, the one dot chain line shows the second line for convenience and further, the arrow shown by the two dot chain line indicates a part of cleavage. The dashed line, one-dot chain line, and two-dot chain line arrow are not related to design of the non-woven fabric material. Furthermore, the back surface drawing of non-woven fabric material according to example-3 is symmetrical to the front surface drawing and is therefore omitted.

The non-woven fabric material according to example-3 is the same as the non-woven fabric material according to example-1 except for the shape, size, and placement of the plurality of vertical emboss and plurality of non-vertical emboss. The non-woven fabric material according to example-3 corresponds to the elastic material according to embodiment 4 described above. In other words, an emboss group is made up of two vertical emboss.

The dimension in the longitudinal direction of the vertical emboss is approximately 1 mm and width is approximately 0.2 mm. The dimension in the longitudinal direction of the non-vertical emboss is approximately 1 mm and width is approximately 0.2 mm. Furthermore, the high density interval of vertical emboss is approximately 0.9 mm and low density interval is approximately 2.9 mm. Furthermore, the interval of non-vertical emboss lined up on the same line is larger than the high density interval of vertical emboss and is approximately 4 mm. Furthermore, the thickness of the non-woven fabric material according to example-3 is 1 mm.

### [Example-4]

Fig. 15 is a front view that shows non-woven fabric material according to example 4 where (a) is a picture of non-woven fabric material prior to performing extension processing and in a loose state and (b) is a picture of non-woven fabric material after performing extension processing and in a stretched state. Note, the dashed line in the front view shows the first line for convenience, the one dot chain line shows the second line for convenience and further, the arrow shown by the two dot chain line indicates a part of cleavage. The dashed line, one-dot chain line, and two-dot chain line arrow are not related to design of the non-woven fabric material. Furthermore, the back surface drawing of non-woven fabric material according to example-4 is symmetrical to the front surface drawing and is therefore omitted.

The non-woven fabric material according to example-4 is the same as the non-woven fabric material according to example-1 except for the shape, size, and placement of the plurality of vertical emboss and plurality of non-vertical emboss. The non-woven fabric material according to example-3 corresponds to the elastic material according to embodiment 5 described above. In other words, being provided with a first emboss group made up of three vertical emboss and a second emboss group being provided with two vertical emboss.

The dimension in the longitudinal direction of the vertical emboss is approximately 1mm and width is approximately 0.2 mm. The dimension in the longitudinal direction of the non-vertical emboss is approximately 1 mm and width is approximately 0.2 mm.

Furthermore, the high density interval of the vertical emboss is approximately 0.9 mm and of the intervals for emboss groups, the wide interval is approximately 2.9 mm and the narrow interval is approximately 2 mm. Furthermore, the thickness of the non-woven fabric material according to example-4 is 1 mm.

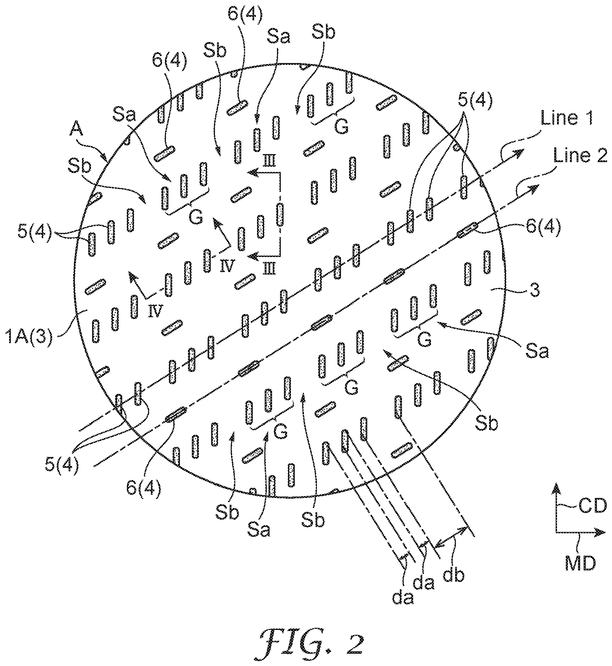

With the example-described above, the direction of the first line and second line intersecting diagonally with regards to the MD direction respectively is as described below. As shown in Fig. 2, Fig. 8 to 10, in state prior to extension processing and stretched (while performing emboss processing on the elastic material manufacturing equipment), this is close to 30 degrees relative to the MD direction. Furthermore, prior to extension processing as in Fig. 12 (a), Fig 13 (a), Fig. 14 (a), Fig. 15 (a) and having been removed from the elastic material manufacturing equipment and loosened, this is roughly 55 degrees relative to the MD direction. Furthermore, after extension processing as in Fig. 12 (b), Fig. 13 (b), Fig. 14 (b) and Fig. 15 (b) and in stretched state (while cleaving), this is roughly 35 degrees relative to the MD direction. Furthermore, after extension processing and in a loosened state after extension processing, this is roughly 60 degrees relative to the MD direction.

### [Comparative Example]

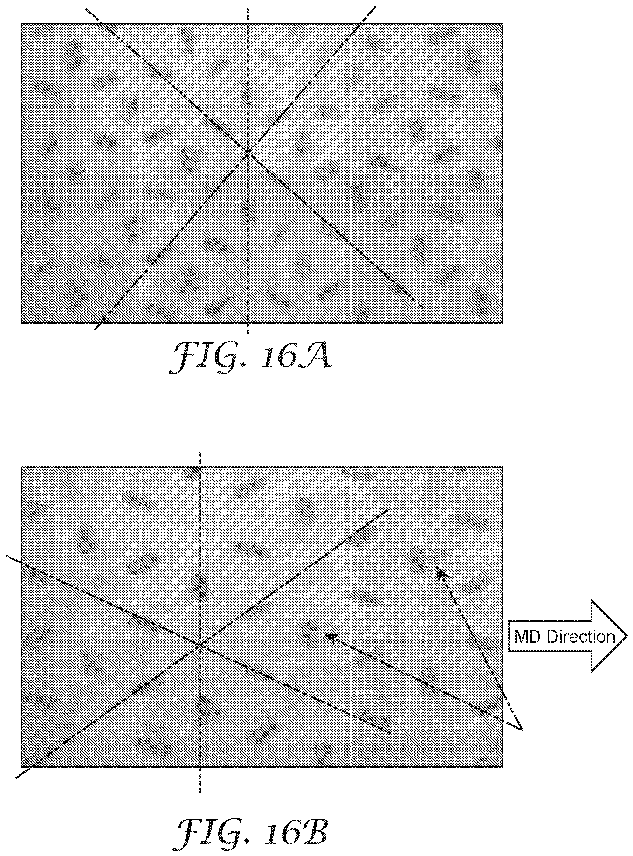

Fig. 16 is a front view that shows non-woven fabric material according to a comparative example where (a) is a picture of non-woven fabric material prior to performing extension processing and in a loose state and (b) is a picture of non-woven fabric material after performing extension processing and in a stretched state.

The non-woven fabric material according to a comparative example is the same as the non-woven fabric material according to example-1 except for the shape, size, and placement of the plurality of vertical emboss and plurality of non-vertical emboss. With regards to the non-woven fabric material according to the comparative example, the vertical emboss is not in a direction that intersects diagonally with the MD direction but is lined up along the CD direction (see dashed line in Fig. 16 (a), (b)) orthogonal to the MD direction. Furthermore, the non-vertical emboss is lined up along a direction diagonally intersecting the MD direction (see the one dot chain line in Fig. 16 (a), (b)). With the non-woven fabric material according to the comparative example, the interval of vertical emboss lined up on the same line is larger than interval of non-vertical emboss lined up on the same line.

The dimension in the longitudinal direction of the vertical emboss is approximately 1 mm and width is approximately 0.3 mm. The dimension in the longitudinal direction of the non-vertical emboss is approximately 1 mm and width is approximately 0.3 mm. Furthermore, the thickness of the non-woven fabric material according to the comparative example is 2.5mm.

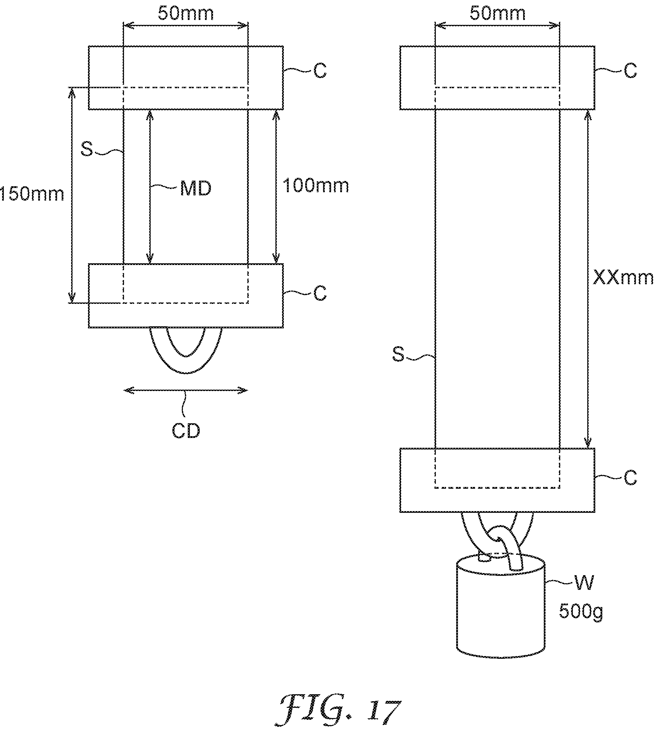

Next, extension processing tests performed with regards to each of the example and the comparative example described above will be described with reference to Fig. 17. This testing used a rectangular sample S respectively for each example-and for the comparative example. For each sample S the dimension in the MD direction is 150 mm and the dimension in the CD direction is 50 mm. For each sample S the upper edge and lower edge were clamped in a chuck C. In a state of suspension without a weight W, the distance between the upper and lower chuck C was 100 mm. With the upper chuck C secured, a weight W of 500 g was suspended from the lower chuck C to perform extension processing. The stretch ratio when the 500 g weight W was suspended is shown in Table 1. For example a stretch ratio of 100% means that the distance between the chuck C went from 100 mm to 200 mm.

**Table 1**

| | Stretch ratio | Elastic stress in MD direction | Stretch in CD direction | Breathability | Gurley stiffness | |
|---|---|---|---|---|---|---|
| | Size | MD: 100 mm | MD: 25 mm | φ 28.6 mm | (mN/cm) | |
| | | CD: 50 mm | CD: 25 mm | | | |
| | % | N/50 mm | mm | Sec/300 ml | MD | CD |
| Example-1 | 100% | 4.8 | 3.8 | 0.3 | 3 | 0.3 |
| Example-2 | 110% | 4.6 | 3.3 | 0.9 | 4.6 | 1.3 |
| Example-3 | 160% | 3.4 | 3.7 | 0.4 | 3.2 | 0.3 |
| Example-4 | 150% | 3.4 | 3.8 | 0.3 | 3.3 | 0.3 |
| Comparative example | 125% | 4.2 | 2 | 1.5 | 4.6 | 1.5 |

As shown in Fig. 12 (b), the non-woven fabric material according to example-1 has cleavage formed in an area along the first line that the vertical emboss is lined up on. In example-1, it can be confirmed that cleavage that is formed between adjacent vertical emboss on the same line aggregate and become a large cleavage.

Furthermore, as shown in Fig. 13 (b), the non-woven fabric material according to example-2 has cleavage formed in an area along the first line that the vertical emboss is lined up on. This cleavage is formed independently between adjacent vertical emboss on the same line.

Furthermore, as shown in Fig. 14 (b), Fig. 15 (b), the non-woven fabric material according to example-3 and example-4 has cleavage formed in an area along the first line that the vertical emboss is lined up on. For this cleavage, it can be confirmed that the cleavage that is formed between adjacent vertical emboss on the same line aggregate and though smaller than that for-example-1, become a large cleavage that is larger than that for example 2.

Furthermore, as shown in Fig. 16 (b), a slight amount of cleavage is formed close to the vertical emboss for the non-woven fabric material according to the comparative example (see the two dot chain arrow of Fig. 16 (b)). Basically, compared to each of the example-described above, the size of the cleavage is small.

Next, breathability tests performed with regards to each of the example-and the comparative example described above will be described. Breathability testing was performed using the air permeability test equipment described below. The results of breathability testing are shown in Table 1.

Gurley densometer manufactured by Toyo Seiki Co., Ltd. (breathability test equipment)
Internal cylinder weight (pressure): 567 g
Air permeability: 300 ml
Transmission surface hole diameter: 28.6 mm (6.45 cm²)

As shown in Table 1, the time required to transmit 300 ml for example-1 is 0.3 seconds. Furthermore, this 0.9 seconds for example-2, 0.4 seconds for example-3, and 0.3 seconds for example-4. On the other hand, this was 1.5 seconds for the comparative example. In other words, each of the examples-were better than the comparative example from the perspective of breathability.

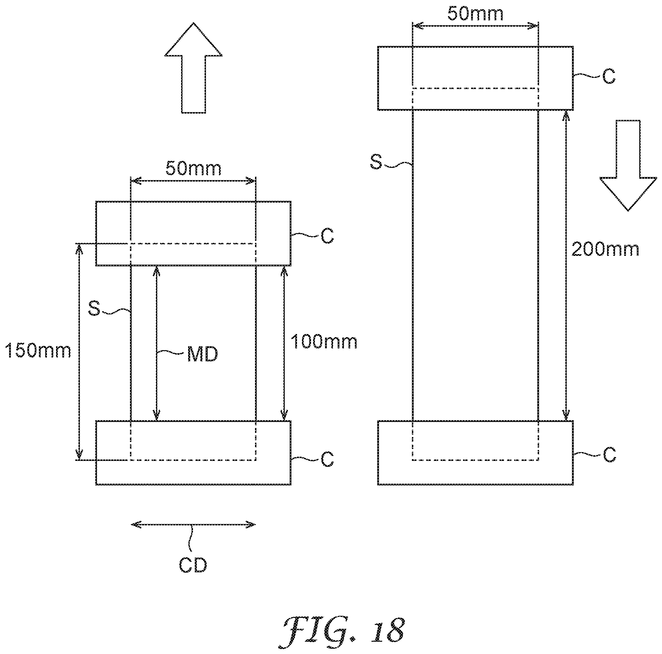

Next, as a result of tension and compression tests performed with respect to each of the examples-and the comparative example described above, the elastic force in the MD direction (MD loading force) will be described with reference to Fig. 18. This testing used a rectangular sample S respectively for each example-and for the comparative example. For each sample S the dimension in the MD direction is 150 mm and the dimension in the CD direction is 50 mm. For each sample S the upper edge and lower edge were clamped in a chuck C. The distance between the upper and lower chuck C is 100 mm. The upper chuck is raised until there is 200 mm between the chuck C and then without stopping, lowered until reaching the original height (1 cycle) and this cycle test was performed. The raise and lower speed of the chuck C was set to 500 mm/m. The tension force at 200 mm between the chuck C was measured. The measurement results are shown in Table 1.

Next, the results of the stretch test performed in the CD direction with regards to each of the example-and the comparative example described above will be described. This testing used a rectangular sample S respectively for each example-and for the comparative example. The dimension of each sample in the MD direction was 25 mm and the dimension in the CD direction was 25 mm. In this state, the amount of extension for a 1 N load in the CD direction was measured. The measurement results are shown in Table 1.

Next, the results of a Gurley stiffness test performed with regards to each of the examples-and the comparative example described above will be described. Gurley stiffness is specified by JIS L 1913 and measures the stiffness when non-woven fabric material or the like is bent from the surface to a vertical direction (vertical direction from either MD direction or CD direction). A low value indicates softness. Gurley stiffness in the MD direction is the results of applying a force in the vertical direction in a line (bending line) that bends in the MD direction. Gurley stiffness in the CD direction is the results of applying a force in the vertical direction in a line (bending line) that bends in the CD direction. A TAPPI T543 om-11 was used for this test. The results of this testing are shown in Table 1.

The Gurley stiffness value of the comparative example in the MD direction is 4.6 mN/cm and the Gurley stiffness in the CD direction is 1.5 mN/cm. On the other hand, the Gurley stiffness in the MD direction for-example-1, example-3, and example-were relatively lower and that for example-2 at least ensured the same level. Furthermore, with regards to the Gurley stiffness in the CD direction, all of the examples-were lower than that for the comparative example.

Bulges between vertical emboss become a mountain, hill, or fold type bulge. This type of bulge causes resistance regarding bending in the vertical direction and in particular, when this bulge part is formed uniform between the vertical emboss, it can cause reduction in softness. However, the non-woven fabric material according to example 1, example-3, and example-4 is prepared with an interval spacing high density areas and interval spacing low density areas where the bulging of a high density area is lower than that of a low density area enabling forming an area where the resistance described above is reduced. As a result, the non-woven fabric material according to example-1, example-3, example-4 is better from the perspective of softness. Furthermore, while being common with example-2, the first line on which a plurality of vertical emboss are lined up on is along a direction diagonally intersecting relative to the MD direction where even if there is a bulge part formed, the bulge part will be formed along a direction intersecting diagonally relative to the MD direction. As a result, this does not readily cause resistance for a position along the MD direction or CD direction and less likely to generate resistance and better from the perspective of softness in the MD direction and CD direction.

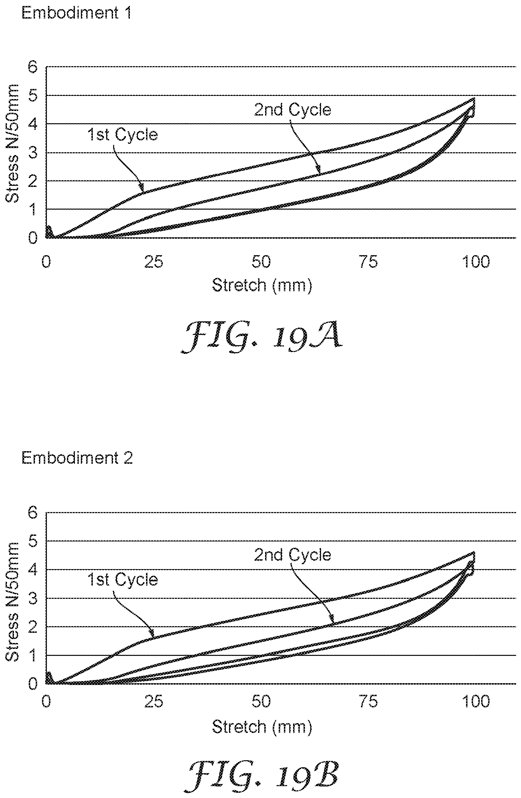

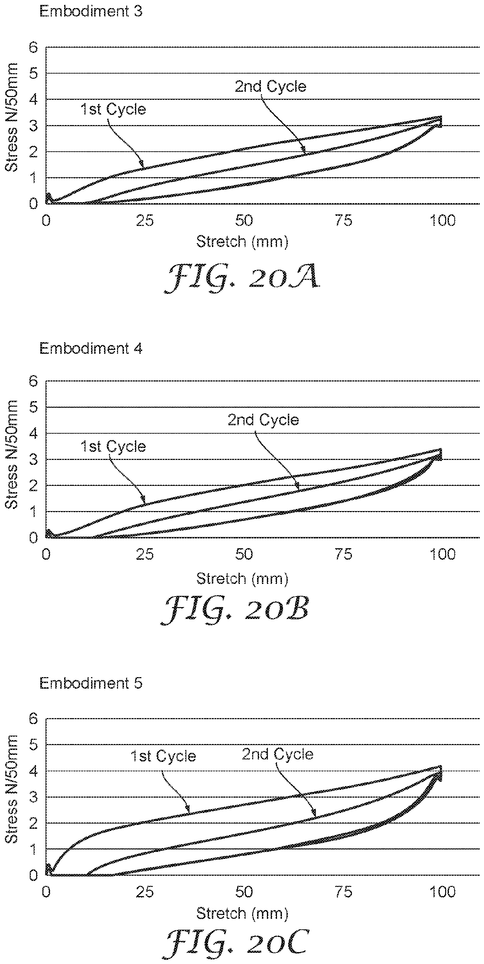

Next, stress characteristic tests performed with regards to each of the example-and the comparative example described above will be described with reference to Fig. 19 and Fig. 20. Fig. 19 and Fig. 20 show an S-S curve obtained for this test. Fig. 19 (a) is the test results for example-1 and Fig. 19 (b) is the test results for example-2. Furthermore, Fig. 20 (a) is the test results for example-3, Fig. 20 (b) is the test results for example-4, and Fig. 20 (c) is the test results for the comparative example.. Note, two cycles were performed for the test where a sample without cleavage where cleavage was formed through applying stress was used for the first cycle and the S-S curve generated and the second cycle was applying stress to a sample where cleavage had been formed and the S-S curve generated.

As shown in Fig. 19 and Fig. 20, cleavage was formed for each of the example-but it can be seen that desired elasticity is ensured. In particular, with regards to example-1 or example-2, there is a lot of cleavage as compared to the comparative example enabling high breathability but elasticity equivalent to that of the comparative example is ensured.

### EXPLANATION OF CODES

- 1A, 1B, 1C, 1D, 1E.: Elastic material
- 2.: Elastic film
- 3.: Non-woven fabric
- 4.: Crimp part
- 5.: Vertical emboss (first emboss)
- 6.: Non-vertical emboss (second emboss)
- 7.: Cleavage
- 10A, 10B, 10C: Sanitary articles
- 11.: Main body part
- 11a.: Abdomen part
- 11b: Back part
- 12.: Waist opening
- 13.: Leg opening part
- 15A, 15B, 15C: Elastic part
- Sa.: High density area
- Sb.: Low density area

## Claims

1. An elastic material (1A, 1B, 1C, ID, 1E) comprising
an elastic film (2),
non-woven fabric (3) laminated on both surfaces of the elastic film (2),
and crimped parts (4) where the non-woven fabric (3) and elastic film (2) are crimped and indented,
the crimped part (4) is prepared with a plurality of first emboss (5) formed on a first line and a plurality of second emboss (6) formed on a second line beside the first line, the first line and the second line in a direction diagonally intersecting the MD direction of the elastic film (2),
wherein the first emboss (5) is a long lengthy shape in a direction orthogonal to the MD direction,
the first line and second line are alternately arranged,
at least a part of the intervals (da, db) of adjacent first emboss (5) on the same line are smaller than the intervals of adjacent second emboss (6) on the same line.

2. The elastic material (1A, 1B, 1C, ID, 1E) according to claim 1, wherein the plurality of first emboss (5) on the same line are arranged at equidistant intervals.

3. The elastic material (1A, 1B, 1C, ID, 1E) according to claim 1, wherein the plurality of first emboss (5) on the same line are at non-equidistant intervals (da, db) with interval spacing high density areas (Sa) and
interval spacing low density areas (Sb) being formed.

4. The elastic material (1A, 1B, 1C, ID, 1E) according to claim 3, wherein there are differing types of numbers of first emboss (5) for the high density areas (Sa).

5. The elastic material (1A, 1B, 1C, ID, 1E) according to anyone of claims 1 to 4, wherein the second emboss (6) is a long and lengthy shape that intersects diagonally with the MD direction.

6. An elastic material (1A, 1B, 1C, ID, 1E) according to claim 1, wherein cleavage is formed on the elastic film (2) on at least a part of the space between adjacent first emboss (5) on the same line.

7. The elastic material (1A, 1B, 1C, ID, 1E) according to claim 6, wherein the plurality of first emboss (5) on the same line are at non-equidistant intervals (da, db) with interval spacing high density areas (Sa) and interval spacing low density areas (Sb) being formed.

8. The elastic material (1A, 1B, 1C, ID, 1E) according to claim 6 or 7, wherein the second emboss (6) is a long and lengthy shape that intersects diagonally with the MD direction.

9. An underpants type sanitary article (10A, 10B, 10C) comprising,
a main body part (11) with an abdomen part (11a) and back part (11b) with both parts (11a, 11b) connected, the main body part (11) being provided with a waist opening part (12), the main body part (11) being provided with a pair of leg openings (13),
and the main body part (11) being provided with an elastic part (15A, 15B, 15C), wherein the elastic part (15A, 15B, 15C) comprises an elastic film (2) according to claim 1.

10. The sanitary article (10A, 10B, 10C) according to claim 9, wherein at least a part between adjacent first emboss (5) on the same line have cleavage (7) formed in the elastic film (2).

## Patentansprüche

1. Ein elastisches Material (1A, 1B, 1C, 1D, 1E), umfassend eine elastische Folie (2), Vliesstoff (3), der auf beide Oberflächen der elastischen Folie (2) laminiert ist, und gekräuselte Teile (4), wo der Vliesstoff (3) und die elastische Folie (2) gekräuselt und eingezogen sind,
wobei der gekräuselte Teil (4) mit einer Mehrzahl von ersten Prägungen (5) hergestellt ist, die auf einer ersten Linie gebildet sind, und einer Mehrzahl von zweiten Prägungen (6), die auf einer zweiten Linie neben der ersten Linie gebildet sind, wobei die erste Linie und die zweite Linie in einer Richtung die MD-Richtung der elastischen Folie (2) diagonal schneiden,
wobei die erste Prägung (5) eine lange längliche Form in einer Richtung orthogonal zur MD-Richtung ist,
die erste Linie und die zweite Linie abwechselnd angeordnet sind,
mindestens ein Teil der Intervalle (da, db) benachbarter erster Prägungen (5) auf derselben Linie kleiner als die Intervalle benachbarter zweiter Prägungen (6) auf derselben Linie sind.

2. Das elastische Material (1A, 1B, 1C, 1D, 1E) nach Anspruch 1, wobei die Mehrzahl von ersten Prägungen (5) auf derselben Linie in äquidistanten Intervallen angeordnet ist.

3. Das elastische Material (1A, 1B, 1C, 1D, 1E) nach Anspruch 1, wobei die Mehrzahl von ersten Prägungen (5) auf derselben Linie in nicht äquidistanten Intervallen (da, db) liegt, wobei Intervallabstandsbereiche hoher Dichte (Sa) und Intervallabstandsbereiche niedriger Dichte (Sb) ausgebildet sind.

4. Das elastische Material (1A, 1B, 1C, 1D, 1E) nach Anspruch 3, wobei es unterschiedliche Arten von Anzahlen von ersten Prägungen (5) für die Bereiche hoher Dichte (Sa) gibt.

5. Das elastische Material (1A, 1B, 1C, 1D, 1E) nach einem der Ansprüche 1 bis 4, wobei die zweite Prägung (6) eine lange und längliche Form ist, die sich diagonal mit der MD-Richtung schneidet.

6. Das elastische Material (1A, 1B, 1C, 1D, 1E) nach Anspruch 1, wobei eine Spaltung auf der elastischen Folie (2) auf mindestens einem Teil des Raums zwischen benachbarten ersten Prägungen (5) auf derselben Linie ausgebildet ist.

7. Das elastische Material (1A, 1B, 1C, 1D, 1E) nach Anspruch 6, wobei die Mehrzahl von ersten Prägungen (5) auf derselben Linie in nicht äquidistanten Intervallen (da, db) liegt, wobei Intervallabstandsbereiche hoher Dichte (Sa) und Intervallabstandsbereiche niedriger Dichte (Sb) ausgebildet sind.

8. Das elastische Material (1A, 1B, 1C, 1D, 1E) nach Anspruch 6 oder 7, wobei die zweite Prägung (6) eine lange und längliche Form ist, die sich diagonal mit der MD-Richtung schneidet.

9. Ein Hygieneartikel vom Unterhosentyp (10A, 10B, 10C) umfassend,
einen Hauptkörperteil (11) mit einem Bauchteil (11a) und einem rückseitigen Teil (11b), wobei beide Teile (11a, 11b) verbunden sind, der Hauptkörperteil (11) mit einem Taillenöffnungsteil (12) versehen ist, der Hauptkörperteil (11) mit einem Paar Beinöffnungen (13) versehen ist,
und wobei der Hauptkörperteil (11) mit einem elastischen Teil (15A, 15B, 15C) versehen ist, wobei der elastische Teil (15A, 15B, 15C) eine elastische Folie (2) nach Anspruch 1 umfasst.

10. Der Hygieneartikel (10A, 10B, 10C) nach Anspruch 9, wobei mindestens ein Teil zwischen benachbarten ersten Prägungen (5) auf derselben Linie eine Spaltung (7) aufweist, die in der elastischen Folie (2) ausgebildet ist.

## Revendications

1. Matériau élastique (1A, 1B, 1C, 1D, 1E) comprenant un film élastique (2), une étoffe non tissée (3) stratifiée sur l'une et l'autre des surfaces du film élastique (2), et des parties crêpées (4) où l'étoffe non tissée (3) et le film élastique (2) sont crêpés et échancrés,
la partie crêpée (4) est préparée avec une pluralité de premiers gaufrages (5) formés sur une première ligne et une pluralité de deuxièmes gaufrages (6) formés sur une deuxième ligne à côté de la première ligne, la première ligne et la deuxième ligne dans une direction croisant en diagonale la direction machine du film élastique (2),
dans lequel le premier gaufrage (5) est une forme allongée longue dans une direction orthogonale à la direction machine,
la première ligne et la deuxième ligne sont agencées en alternance,
au moins une partie des intervalles (da, db) de premiers gaufrages adjacents (5) sur la même ligne sont plus petits que les intervalles de deuxièmes gaufrages (6) adjacents sur la même ligne.

2. Matériau élastique (1A, 1B, 1C, 1D, 1E) selon la revendication 1, dans lequel la pluralité de premiers gaufrages (5) sur la même ligne sont agencés à des intervalles équidistants.

3. Matériau élastique (1A, 1B, 1C, 1D, 1E) selon la revendication 1, dans lequel la pluralité de premiers gaufrages (5) sur la même ligne sont à des intervalles non équidistants (da, db) avec des zones à forte densité d'espacements d'intervalles (Sa) et des zones à faible densité d'espacements d'intervalles (Sb) étant formées.

4. Matériau élastique (1A, 1B, 1C, 1D, 1E) selon la revendication 3, dans lequel il y a différents types de nombres de premiers gaufrages (5) pour les zones à forte densité (Sa).

5. Matériau élastique (1A, 1B, 1C, 1D, 1E) selon l'une quelconque des revendications 1 à 4, dans lequel le deuxième gaufrage (6) est une forme longue et allongée qui croise en diagonale la direction machine.

6. Matériau élastique (1A, 1B, 1C, 1D, 1E) selon la revendication 1, dans lequel un clivage est formé sur le film élastique (2) sur au moins une partie de l'espace entre des premiers gaufrages adjacents (5) sur la même ligne.

7. Matériau élastique (1A, 1B, 1C, 1D, 1E) selon la revendication 6, dans lequel la pluralité de premiers gaufrages (5) sur la même ligne sont à des intervalles non équidistants (da, db) avec des zones à forte densité d'espacements d'intervalles (Sa) et des zones à faible densité d'espacements d'intervalles (Sb) étant formées.

8. Matériau élastique (1A, 1B, 1C, 1D, 1E) selon la revendication 6 ou 7, dans lequel le deuxième gaufrage (6) est une forme longue et allongée qui croise en diagonale la direction machine.

9. Article hygiénique de type culotte (10A, 10B, 10C) comprenant,
une partie de corps principal (11) avec une partie d'abdomen (11a) et une partie de dos (11b) avec l'une et l'autre des parties (11a, 11b) reliées, la partie de corps principal (11) étant pourvue d'une partie d'ouverture de taille (12), la partie de corps principal (11) étant pourvue d'une paire d'ouvertures de jambe (13),
et la partie de corps principal (11) étant pourvue d'une partie élastique (15A, 15B, 15C), dans lequel la partie élastique (15A, 15B, 15C) comprend un film élastique (2) selon la revendication 1.

10. Article hygiénique (10A, 10B, 10C) selon la revendication 9, dans lequel au moins une partie entre des premiers gaufrages adjacents (5) sur la même ligne a un clivage (7) formé dans le film élastique (2).
